Europäisches Patentamt

European Patent Office

Office européen des brevets

(11) Publication number: **0 420 171 A1**

## EUROPEAN PATENT APPLICATION

(21) Application number: 90118445.7

(22) Date of filing: 26.09.90

(51) Int. Cl.5: **C12N 5/00**

(30) Priority: 26.09.89 JP 249443/89
12.09.90 JP 242246/90

(43) Date of publication of application:
**03.04.91 Bulletin 91/14**

(84) Designated Contracting States:
**CH DE DK FR GB LI NL SE**

(71) Applicant: **KIRIN BEER KABUSHIKI KAISHA**
**26-1 Jingumae 6-chome**
**Shibuya-ku Tokyo-To(JP)**

(72) Inventor: **Ogata, Masafumi, c/o Kirin Beer**
Kabushiki Kaisha
26-1, Jingumae 6-chome, Shibuya-ku
Tokyo-to(JP)
Inventor: **Matsukura, Hideki, c/o Kirin Beer**
**Kabushiki Kaisha**
26-1, Jingumae 6-chome, Shibuya-ku
Tokyo-to(JP)

(74) Representative: **Reichel, Wolfgang, Dipl.-Ing.**
**et al**
**Reichel und Reichel Parkstrasse 13**
**W-6000 Frankfurt am Main 1(DE)**

(54) Carrier for culturing animal cells and a process for preparing it.

(57) A carrier for culturing animal cells comprises a ground matrix having a three-dimensional network structure and a fibrous protein for coating the surface of the ground matrix. The cells can adhere to the interior of the carrier, and thus the carrier enables cell culture in high concentrations.

FIG. 3

EP 0 420 171 A1

EP 0 420 171 A1

## CARRIER FOR CULTURING ANIMAL CELLS AND A PROCESS FOR PREPARING IT

### BACKGROUND OF THE INVENTION

Field of the Invention

The present invention relates to a carrier for culturing animal cells and a process for preparing it.

Description of Related Art

As one of the methods for culturing animal cells, a culturing method with use of a microcarrier has hitherto been tried.

The carrier used for this culturing method has generally a particle diameter in the range of 100 - 230 $\mu$m (wet) and a specific gravity in the range of 1.030 -1.045, and is formed into beads. As the material of the carrier, there are used gelatin, polyacrylamide, dextran, cellulose, polystyrene, collagen and the like. These beads are optionally subjected to surface treatments such as coating with collagen or the application of surface charge.

The microcarriers are charged into a tank and carry animal cells on their surfaces for the production of interferon, monoclonal antibodies or a variety of viruses.

These conventional microcarriers however tend to cause problems of adherence or cell growth properties when used as large scale culturing carrier of anchorage dependent cells. This is because the microcarriers have adherence or growth properties of cells whose properties vastly vary with the cell lines. Since cells grow only on the surface of a conventional carrier, in order to increase the adhering area of the cells the particle diameter must be minimized within the permittable range or the concentration of the carrier must be increased. However, it is difficult to separate the carrier from a culturing medium with these techniques, which require a specific devices. If the carrier concentration is increased, the carriers are apt to collide with each other and impair significantly adherent cells to cause serious problem for their use on an industrial scale. Thus, it is considered that the cell concentration has a threshold in $1 \times 10^6$ to $5 \times 10^6$ cells/ml for the usual culturing of cells.

There are additional various problems such as the microcarrier itself being very expensive or unsuitable for industrial production from an economical reason.

### SUMMARY OF THE INVENTION

Accordingly, the object of the present invention is to provide a carrier for culturing animal cells which is excellent in the adhesion of animal cells and thus enables cell culture in high concentrations.

The carrier for culturing animal cells according to the present invention comprises a ground matrix having a three-dimensional network structure and a fibrous protein for coating the surface of the ground matrix.

According to a preferable embodiment of the carrier according to the present invention, the ground matrix comprises a foam having a void of 80% and an average pore size of 0.05 to 2.0 mm.

The carrier for culturing animal cells according to the present invention has a three-dimensional network structure, and the cells can adhere to not only to the surface of the carrier but also its interior (the adhesion of cells probably occurs substantially at the interior of the carrier according to the present invention). Thus, the cell adhesion area increases remarkably as compared with the conventional carrier in the shape of beads of which only the external surface cells adhere to. The three-dimensional network structure prompts the material displacement within the ground matrix. For example, the nutriment in a culture medium easily diffuses into the interior of the carrier and the products and inhibitors such as ammonia or lactic acid are also excreted from the carrier with ease. Cells can be cultured even at the interior of the carrier. Collision between the carriers will not cause the serious impairment of the animal cells which are mostly present in the interior of the carrier, so that the concentration of the carrier can be increased (to an extent of 50 to 60% increase of carrier concentration based on the apparent volume).

2

In addition, the adhesion of the cells is increased since the surface of the carrier is coated with a fibrous protein. The adhesion of the cells is maintained over a long period of time since the fibrous protein is optionally insolubilized by crosslinking.

## BRIEF DESCRIPTION OF THE DRAWINGS

Fig. 1 is a schematic perspective view which illustrates an example of the carrier for culturing animal cells according to the present invention,

Fig. 2 is a partial enlarged sectional view of the perspective,

Fig. 3 is an enlarged photograph at 148 magnification of the carrier,

Fig. 4 is an enlarged photograph at 300 magnification of the carrier,

Fig. 5 is a graph which illustrates the variation with the passage of time of the cell concentration, the glucose concentration and the viability when the culturing was conducted with the carrier according to the present invention,

Fig. 6 is a diagram which illustrates an apparatus for applying the carrier to a fluidized bed apparatus,

Fig. 7 is a graph which illustrates the variation with the passage of time of the cell concentration and the glucose concentration when the culturing was conducted with the apparatus, and

Figs. 8 - 10 is the enlarged photographs of the carriers for culturing animal cells according to the present invention in which the CHO-K1 cells, the HeLa cells and the L929 cells, respectively, adhere to the interior thereof.

## DETAILED DESCRIPTION OF THE INVENTION

The present invention is described in the following examples with reference to the drawings.

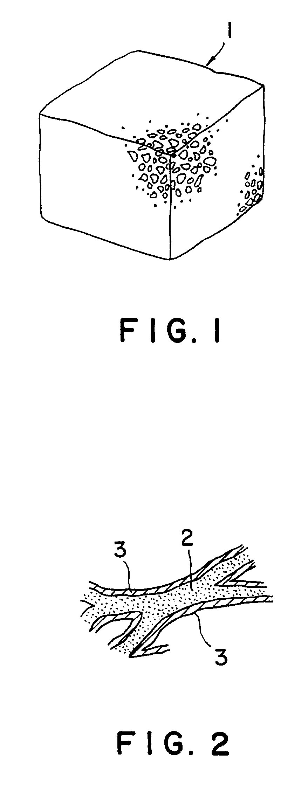

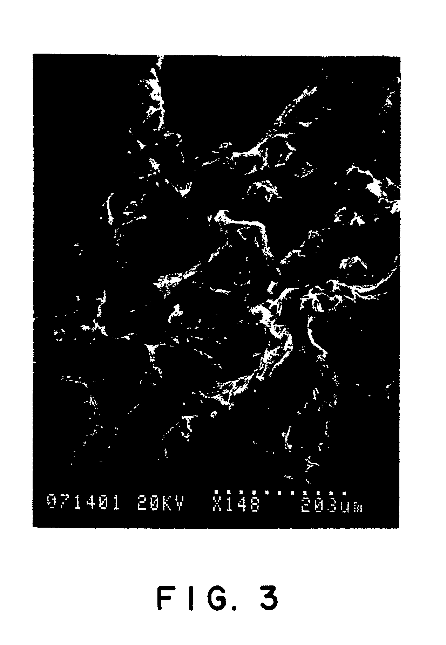

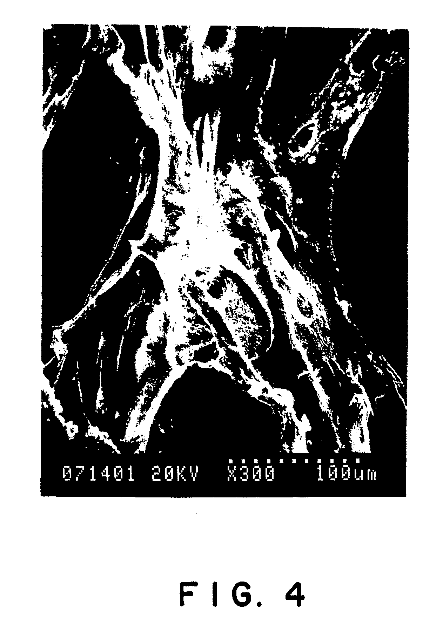

The carrier for culturing animal cells 1 according to the present invention is illustrated as a partial enlargement in Fig. 1 and, as shown as a partial enlargement of it in Fig. 2, has a structure in which the surface of a ground matrix 2 having a three-dimensional structure is coated with a fibrous protein 3 which has been optionally insolubilized by crosslinking treatment (see photographs in Figs. 3 and 4 as for the actual structural forms).

The term "three-dimensional network structure" herein means a structure having continuous pores which permits the culture of animal cells in the inside of the pores.

According to a preferable embodiment of the present invention, the ground matrix comprises a foam having a void of at least 80%, preferably at least 95%, more preferably at least 97%, and an average pore size of 0.05 to 2.0 mm, preferably 0.1 to 1.5 mm. When the void and the average pore size of the ground matrix are present within the aforementioned ranges, a nutrition source can be supplied sufficiently into the inside of the carrier and thus cells even in the depth of the carrier will not cause necrosis. On the other hand, when the average pore size is 0.03 mm or less, many of the animal cells in the inside of the carrier are undesirably incapable of substantial culturing

The carrier for culturing animal cells according to the present invention comprises coating the surface of the aforementioned ground matrix with a fibrous protein. In this connection, the coating layer of the fibrous protein has a very small thickness, so that the void and the average pore size of the above-described ground matrix correspond substantially to those of the carrier.

The ground matrix comprises preferably a foam of a natural polymer such as cellulose. The cellulose may be either a natural cellulose, i.e. crystalline form I or a regenerated cellulose, i.e. crystalline form II. It will be advantageous to use as the ground matrix an elastic material such as a foam, since it enables a further reduction of the effect of the collision of the carriers on the cells.

The cellulose foam which is suitable for use as the ground matrix is prepared by subjecting a high purity pulp made of wood to chemical treatment to form a viscose, to which a third component for forming pores is then added, mixing the blend, injecting it into a mold, solidifying it and appropriately removing the third component. As the third component in this method, there are used materials which can be easily removed after forming a foam, such as crystalline materials insoluble or sparingly soluble in aqueous solutions, waxes, surfactants having a low HLB value, sublimate substances and the like. The third component is removed by an appropriate method selected from extraction with an organic solvent, sublimation by heating or the like depending on the properties of the third components. According to this method, a ground matrix having desirable physical properties such as voids or pore size is easily prepared by appropriately selecting the size, kind and amount of the third component to be incorporated.

In the most preferable embodiment, the ground matrix comprises a cellulose foam having a void of at least 97%, an average pore size of 0.5 to 1.5 mm and a specific gravity of 1.4 to 1.7 $g/cm^3$.

The void and average pore size of the ground matrix can be determined by the methods of mercury injection and BET such as described in "POROUS MATERIAL HANDBOOK", ed. Jun Kamizawa and Masanobu Kakeya, published by IPC (1988) Tokyo, Japan.

The carrier for culturing animal cells according to the present invention is the above-described ground matrix of which the surface has been coated with a fibrous protein.

As the fibrous protein, collagen or gelatine is preferably used from the viewpoint of the adhesion of cells. Some of the fibrous proteins are advantageously subjected to insolubilization treatment such as crosslinking.

The coating of the ground matrix with the fibrous protein is generally conducted by dipping the ground matrix in the fibrous protein and then drying the protein. The coating is preferably carried out by the following procedure. First, the ground matrix is dipped into a solution of, for example, an acid solubilized collagen or an enzyme solubilized collagen and then air-dried at room temperature to form a coat on the surface of the ground matrix. The collagen solution has a concentration of 0.001 to 1.0% (w/v), preferably 0.01 to 0.5% (w/v) and an appropriate pH in the range of 3.0 to 7.0.

The insolubilization of the protein can be conducted by the treatment with crosslinking agents such as glutaraldehyde or hexamethyleneisocyanate or the irradiation of UV rays or $\gamma$ rays. With the multivalent reagents, crosslinking is conducted by dissolving the reagent into a solution such as a 0.05 to 1.0 M phosphate buffer to ensure that the concentration is in the range of 0.1 to 10% (w/v), dipping the carrier into the solution and shaking the mixture for from 10 minutes to 2 hours. With the irradiation of UV rays, crosslinking is conducted at room temperature for from 30 minutes to 12 hours.

The crosslinked carrier thus obtained is rinsed with plenty of a solution such as a 0.05 to 1.0 M phosphate buffer and equilibrated with the buffer to give a carrier for culturing cells.

In order to improve the adhesion of cells, it is also possible to incorporate a cell adhesion promoting glycosilated proteins such as fibronectin or vitronectin into collagen for reinforcement.

When a fibrous protein is bonded to a ground matrix consisting of cellulose, it is also preferable to have the cellulose ground matrix activated preliminarily with any one of a variety of appropriate methods such as activation with cyanogen bromide, epoxide activation or periodic acid activation.

The carrier according to the present invention is uses as a molded form having a shape and a size depending on various culturing methods. The aforementioned carrier 1 is preferably formed into an appropriate form such as a cube, a rectangular parallelepiped or a sphere. When the ground matrix is a foam, the carrier according to the present invention is generally obtained as small pieces which are made by chopping the foam. The ground matrix may be coated with the aforementioned fibrous protein either before or after the ground matrix has been chopped into small pieces. However, it is preferable to conduct coating after chopping the ground matrix since the coating can be conducted more effieciently.

The carrier according to the present invention has an excellent adhesion and proliferation properties to many adherent animal cell lines. It is thus possible to use it as a carrier for culturing a wide range animal cells. Specific examples of animal cell lines include fibroblasts such as human embryo preputial fibroblast, Chinese hamster lung fibroblast, hen embryo fibroblast and Syrian hamster neonatal fibroblast; epithelial cells such as human cervical carcinoma cell, Chinese hamster ovarium cell, mouse matocarcinoma cell, African green monkey renal cancer cell, and vascular endothelial cell.

The carrier for culturing animal cells according to the present invention can be used in the same manner as the conventional carriers, and can perform cultivation at higher concentrations and larger scales when compared with the conventional carriers. For instance, according to the carrier of the present invention, it is possible to realize the cell concentrations of up to $1 \times 10^7$ cell/ml.

Furthermore, the carrier for culturing animal cells according to the present invention can be used for the culture of not only anchorage dependent cell lines but also anchorage independent cell lines such as hybridoma cells.

The present invention is further explained with reference to the Examples.

Example 1

(1) Preparation of carrier for culturing animal cells

EP 0 420 171 A1

As a ground matrix having a three-dimensional network structure, a cellulose foam having a net specific gravity of 1.52 g/cm$^3$, a void of 97% and an average pore size of 1.05 mm (manufactured by SAKAI ENGINEERING K.K., Fukui, JAPAN) was used, and it was chopped into a piece having a size of 1.5 mm × 1.5 mm. The cellulose form was suspended into a 0.05% (w/v) collagen solution (manufactured by KOKEN K.K., bovine calf skin solubilized collagen, type I, pH 3.0). After 1 hour during which the ground matrix had been sufficiently impregnated with the collagen, the ground matrix was placed on a dish and air-dried in an atmosphere at 37°C overnight. Next the air-dried ground matrix was dipped into a 1.0% (w/v) glutaraldehyde solution in 0.1 M phosphate buffer, pH 7.0, and the mixture was stirred by a stirrer at 50 rpm for 2 hours to conduct crosslinking reaction. The reaction was rinsed with a plenty of 0.1 M phosphate buffer, pH 7.0, and equilibrated overnight.

(2) Culturing of Cells

Into a 500 ml spinner flask were placed 100 ml (apparent volume) of the carrier obtained by the aforementioned equilibration and 50 ml of PBS (Ca$^{2+}$-, Mg$^{2+}$-, pH 7.0), and the content was sterilized at 120°C for 15 minutes, rinsed with portions of 50 ml of a DMEM culture medium free of a fetal bovine serum (FBS) and equilibrated with 200 ml of a DMEM culture medium containing a 5% FBS at 37°C under an atmosphere of 95% of air and 5% of CO$_2$.

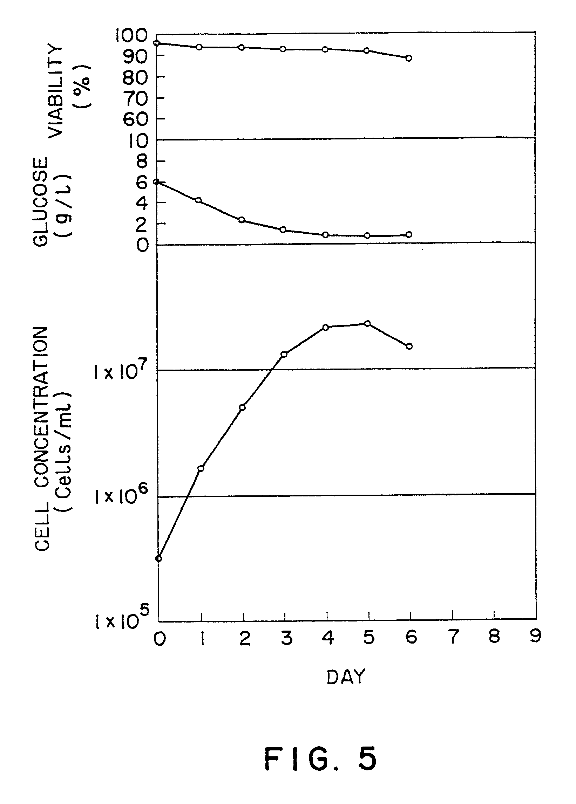

As the cell line, a CHO-K1 strain was employed. It was inoculated at a concentration of 3.0 × 10$^5$ cells/ml. In order to preferably conduct inoculation, 1 minute intermittent stirring (50 rpm) at intervals of 30 minutes was conducted at a stirring speed of 50 rpm under an atmosphere of 95% of air and 5% of CO$_2$. Fig 5 shows the results of the cell concentration, the viablity and the results of the cell concentration, the viability and the variation of glucose concentration.

As apparent from this figure, the viability and glucose concentration of the cell can be stably maintained, and the concentration of the cell exceeded 1 × 10$^7$ in 5 days.

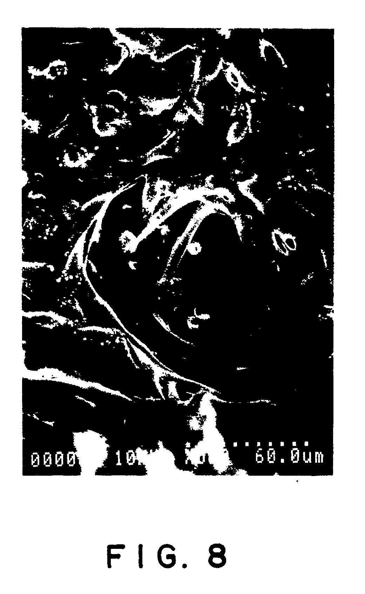

After 2 days form initiating the culturing CHO-K1 cells were observed with a scanning electron microscope. The photograph of the cells were shown in Fig. 8. It can be found from these photographs that the CHO-K1 cells are proliferating successfully in the interior of the carrier.

Example 2

The carrier prepared in Example 1(1) was charged into a fluidized bed reactor 4, and the CHO-K1 strain was cultured. A container having an internal diameter of 20 mm and a height of 70 mm (net volume 22 cm$^3$) was used as the reactor 4, into which 15 cm$^3$ (apparent volume) of the carrier was charged ad the culture medium was supplied at a linear velocity of 4.5 cm/min from a culture medium supply tank 5 to fluidize the contents. A DMEM culture medium containing a 5% fetal bovine serum was employed as a culture medium, and the inlet and outlet speeds of the culture medium were set at 50 cm$^3$/day. The culturing was conducted batchwise for one week from the initiation of the culture and then continued in a continuous operation. In the figure, P illustrates a pump, 6 illustrates a harvest tank, 7 illustrates gas (air, O$_2$ and CO$_2$) tanks, 8 illustrates a fermentor, and 9 illustrates a stirring blade.

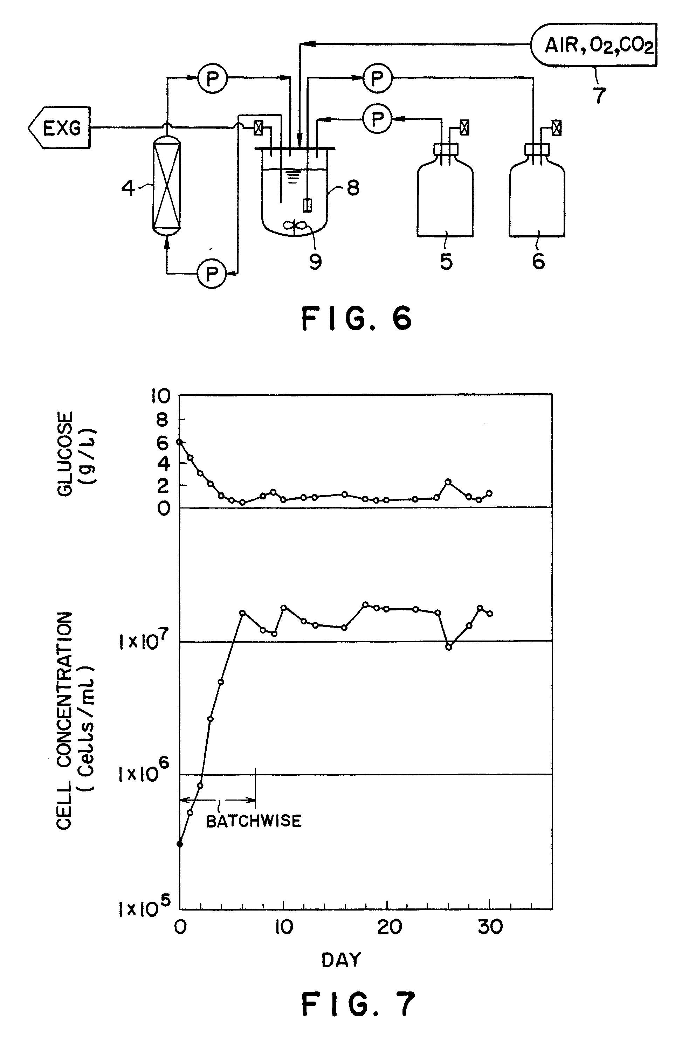

The culturing has been successfully conducted, as is shown in Fig. 7, with maintaining the cell concentration of 5 × 10$^6$ cells/ml after one week of culturing.

Example 3

An epithilial cell, that is a human cervical carcinoma cell line (HeLa) and a fibroblast that is a mouse connective tissue cell line (L929) were cultured in a spinner flask in the same manner as in Example 1.

A DMEM culture medium containing 5% fetal bovine serum was employed as a culture medium with the same culturing operation as Example 1.

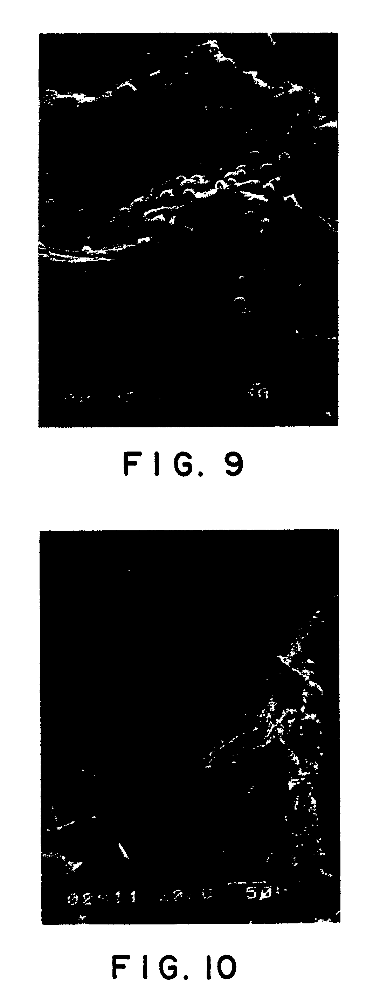

Two days after initiation of culturing, the HeLa cell and the L929 cell were observed with a scanning electrn microscope. The photographs of these cells are shown in Figs. 9 and 10. It can be found from these photographs that the HeLa cells and the L929 cells grow successfully in the interior of the carrier in the same manner as CHO-K1 cells.

It was also confirmed that the cell concentrations at 4 days and 6 days after the initiation of the culturing, as shown in Table 1, reaches at least 5 × 10$^6$ cells/ml.

5

EP 0 420 171 A1

Table 1

| Variation of the cell concentration with the passage of time | | |
|---|---|---|
| Culturing period (day) | HeLa | L929 |
| 0 | $3.0 \times 10^5$ | $3.0 \times 10^5$ |
| 2 | $2.2 \times 10^6$ | $1.5 \times 10^6$ |
| 6 | $9.7 \times 10^6$ | $6.8 \times 10^6$ |

**Claims**

1. A carrier for culturing animal cells comprising a ground matrix having a three-dimensional network structure and a fibrous protein for coating the surface of said ground matrix.

2. A carrier for culturing animal cells according to claim 1, wherein said ground matrix comprises a foam having a void of at least 80% and an average pore size of 0.05 to 2.0 mm.

3. A carrier for culturing animal cells according to claim 1, wherein said ground matrix comprises a foam of a natural polymer.

4. A carrier for culturing animal cells according to claim 3, wherein said ground matrix comprises a cellulose foam.

5. A carrier for culturing animal cells according to claim 4, wherein said ground matrix has a particle diameter of 1.0 to 5.0 mm, a void of at least 97%, an average pore size of 0.5 to 2.0 mm and a specific gravity of 1.4 to 1.7 g/cm³.

6. A carrier for culturing animal cells according to claim 1, wherein said fibrous protein is a protein which is insolubilized by crosslinking treatment.

7. A carrier for culturing animal cells accoring to claim 6, wherein said fibrous protein comprises collagen or gelatin.

8. A process for preparing a carrier for culturing animal cells comprising a steps of providing a ground matrix having a three-dimensional network structure and coating the surface of said ground matrix with a fibrous protein.

6

F I G. I

F I G. 2

F I G. 3

# F I G. 4

FIG. 5

FIG. 6

FIG. 7

FIG. 8

FIG. 9

FIG. 10

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 81, no. 5, 5th August 1974, page 287, abstract no. 23770n, Columbus, Ohio, US; J. LEIGHTON: "Collagen-coated cellulose sponge", & TISSUE CULT.: METHODS APPL. 1973, 367-71 * The entire abstract * | 1-5,7,8 | C 12 N 5/00 |
| X | PATENT ABSTRACTS OF JAPAN, vol. 13, no. 208 (C-596)[3556], 16th May 1989; & JP-A-1 27 464 (CHIYODA CHEM. ENG. & CONSTR. CO., LTD) 30-01-1989 * The entire abstract * | 1,2,5-8 | |

TECHNICAL FIELDS
SEARCHED (Int. Cl.5)

C 12 N

The present search report has been drawn up for all claims

| Place of search | Date of completion of search | Examiner |
|---|---|---|
| The Hague | 14 January 91 | RYCKEBOSCH A.O.A. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document